# EUROPEAN PATENT APPLICATION

(11) **EP 0 629 387 A1**
(43) Date of publication of application: **21.12.1994**
(21) Application number: 94104683.1
(22) Date of filing: 24.03.1994
(51) Int. Cl.: A61F 5/44

(54) **Shaped sanitary towel for incontinence**

(30) Priority: 11.06.1993 IT MI930484 U
(71) Applicant: SILCA S.p.A., I-26017 Trescore Cremasco CR (IT)
(72) Inventor: Arcelloni, Amelio, I-26013 Crema CR (IT); Roscio, Gianmaria, I-22031 Albavilla CN (IT)
(74) Representative: La Ciura, Salvatore

(57) **Abstract**

Shaped sanitary towel for incontinence of the type comprising a back part (5) and a front part (4) connected by a narrower central part (6), where, in correspondence with the front part, a pair of side "wings" (7) is provided which are suitable to come out of the panties and to be seized to correctly place the towel, said wings being located so as to be in correspondence with the patient's groin when the towel is worn, and being interposed between the patient's skin and the rubber band of the panties used to hold the towel in its position.

## Description

The present invention proposes a shaped sanitary towel for incontinence, which is characterized by its peculiar structure, remarkably increasing its practicality of use.

More particularly the object is a sanitary towel to be worn under elastic panties and comprising, in its front part, two side wings coming out of the panties, so as to protect the skin thereby preventing inflammation and ensuring a perfect seal to liquids.

The wings can be seized after the sanitary towel has been worn, in order to place it optimally.

The sanitary towels for incontinence can be divided into two large categories: the panty-towels, which are worn directly and are provided with rubber bands suitable to hold them in their position, and shaped sanitary towels, to be worn under elastic panties having the function of holding them in their position.

The products of the second category are cheaper than the others, because a much smaller quantity of material is needed, which material substantially affects the production cost. On the other hand, it often occurs that these shaped sanitary towels do not provide a perfect seal to liquids due to the difficulties of a correct positioning.

The invention proposes a sanitary towel of the second type, that is to say a shaped sanitary towel to be worn under panties, which combines the advantages of a low production cost with the advantage of a perfect seal, due to the special structure of the parts which allows it to be easily and optimally positioned, so as to avoid the risk of leaks.

Besides, the presence of the wings prevents the rubber band of the panties from coming into contact with the skin causing inflammation and reddening, which on the contrary occurs with the presently known sanitary towels.

The present invention will be now described in detail, as an example, with reference to the enclosed figures where:
Figure 1 illustrates the view from above on the sanitary towel according to the invention, completely open;
Figures 2 and 3 are lateral views of the sanitary towel according to two orthogonal directions.

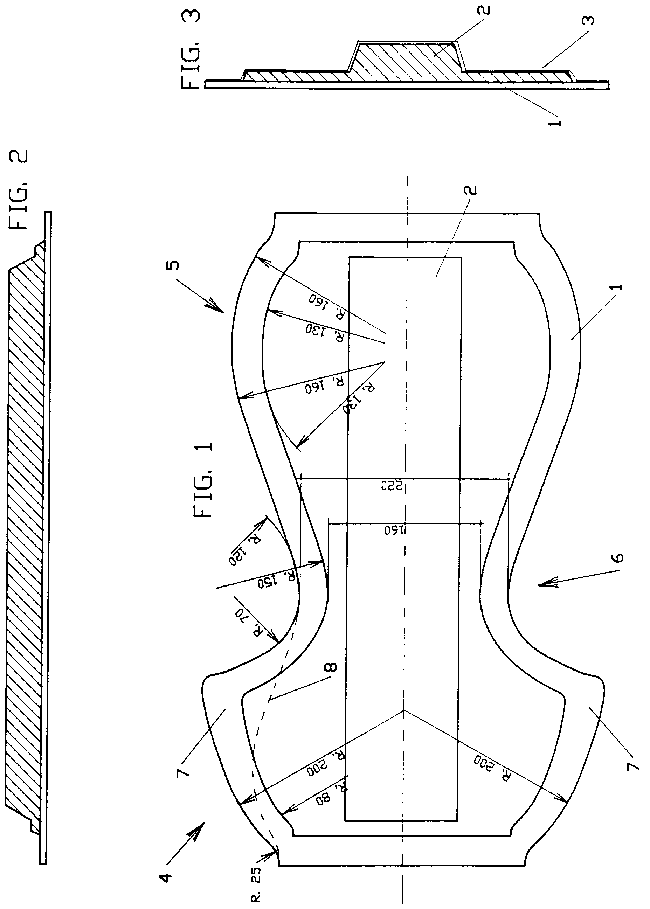

With reference to Figure 3, the sanitary towel according to the invention comprises an outer layer 1 made of an impermeable material, preferably polythene, onto which a fluff or absorbing material 2 is applied, coated with a nonwoven fabric layer 3.

As is clearly visible from the plan view of Figure 1, the sanitary towel is of the shaped type, comprising two enlarged front and back "lobes" or parts, respectively indicated with numerals 4 and 5, connected by a narrower central part, indicated with numeral 6.

The sanitary towel is worn under the panties, which have the function of holding it in a correct position after the parts 4 and 5 have been folded against the patient's skin.

The invention is characterized by the presence, in correspondence with the front part, of a pair of side wings 7 coming out of the outline shaped sanitary towels usually have, which outline is shown with a broken line 8.

When the sanitary towel is worn, these wings 7 are in correspondence with the patient's groin and come out of the panties, allowing on the one hand to seize the sanitary towel to position it correctly in order to ensure a better seal to liquids and, on the other hand, to prevent the rubber band of the panties from coming into contact with the patient's skin, thereby causing inflammation and reddening.

For completeness of description the figure shows possible dimensions of a sanitary towel according to the invention.

It is however clear that these dimension should not be intended as limiting ones, because they can vary according to different parameters and also according to its own size.

With the described solution a shaped sanitary towel is obtained which combines the advantages of cheapness of this type of sanitary towels with the improved functional characteristics of the panty-towels.

The dimensions, as well as the employed materials, can vary according to the intended use.

## Claims

1. A shaped sanitary towel for incontinence of the type comprising larger-sized back part and front part, connected by a narrower central part, characterized in that, in correspondence with the front part, a pair of side wings are provided, said wings being suitable to come out of the panties and to be seized in order to correctly place the sanitary towel.

2. A sanitary towel according to the preceding claim, characterized in that said wings are positioned in such a way as to be in correspondence with the patient's groin when the sanitary towel is worn, said towels being interposed between the patient's skin and the rubber band of the panties used to hold the sanitary towel in its position.
